# EUROPEAN PATENT SPECIFICATION

(11) **EP 2 405 256 B1**
(45) Date of publication and mention of the grant of the patent: **09.01.2019**
(21) Application number: 11173248.3
(22) Date of filing: 08.07.2011
(51) Int. Cl.: G01N 21/64, A61B 5/00

(54) **Quantitative in vivo lifetime imaging using a time-domain platform with a supercontinuum tunable laser for extended spectral coverage**
Quantitative In-vivo-Lebenszeitabbildung mit einer Zeitbereichsplattform mit Superkontinuum-abstimmbarem Laser für erweiterte spektrale Abdeckung
Imagerie de durée de vie in vivo quantitative utilisant une plateforme de domaine temporel avec un laser réglable supercontinuum pour couverture spectrale étendue

(30) Priority: 09.07.2010 US 363005 P
(43) Date of publication of application: 11.01.2012
(73) Proprietor: SoftScan HealthCare Group, Saint-Laurent QC H4S 2A4 (CA)
(72) Inventor: Mincu, Niculae, Quebec H9R 5W8 (CA); Huang, Dao Chao, Montreal, Quebec H1L 5V4 (CA); Piche, Marilyse, Laval, Quebec H7N 2H3 (CA); Ma, Guobin, Dorval, Quebec H9S 2M2 (CA)
(74) Representative: Wittmann, Günther

(56) References cited:
- WO-A1-2005/043138
- WO-A2-2006/091223
- US-A1- 2008 051 665

## Description

### TECHNICAL FIELD

The present disclosure relates to the field of optical imaging of biological tissues. More specifically, the present disclosure relates to a method and a system for collecting optical data for use in time resolved optical imaging of a turbid media. The invention also relates to a quantitative in vivo lifetime imaging using a time-domain platform with a supercontinuum tunable laser for extended spectral coverage.

### BACKGROUND

Different types of imaging techniques such as positron emission tomography (PET), magnetic resonance imaging (MRI) and ultrasound imaging are available that can non-invasively gather information from within biological tissues as a basis for image reconstruction. More recently, another imaging technique, namely optical imaging has been the subject of intense research and commercial development.

Optical imaging is based on information that can be derived from the analysis of the signal resulting from the interaction of light with matter as it is propagated within an object. A time domain (TD) approach, by conveying information on the time required by photons to travel within the object, is considered to be "time resolved" and can be used to calculate the spatial distribution of optical characteristics of the object, such as absorption and scatter coefficients, via well known photon diffusion.

Optical imaging is particularly attractive in view of its non-invasiveness which permits the acquisition of in vivo information without damaging biological tissues. Furthermore the technique may be useful to monitor drug distribution, detect the presence of abnormalities within organs, or map physiological activities within mammals.

Optical imaging systems rely on the presence of bioluminescent molecules, also called biomarkers or fluorescent markers, within a biological region of interest (ROI). For example certain endogenous molecules and some exogenous molecules, such as exogenous chromophores as well as fluorophores, provide useful levels of optical contrast. Recently, an explosive development in the variety of biomarkers and nanoprobes is revolutionizing the molecular imaging and generates new premises for translational medicine paradigm. Molecular imaging using optical methods will be required to provide the means for managing the huge diversity of biomarkers, for their identification and classification, and for a thorough validation of their specificity and reliability.

In view of the above, it would be desirable to provide a method and an optical imaging system for imaging turbid media such as biological tissues that can rapidly and efficiently support a wide variety of fluorescent markers.

### SUMMARY

According to the present disclosure, there is provided a method for collecting optical data for use in time resolved optical imaging of a turbid media. An excitation wavelength of a pulsed light beam is tuned according to an excitation spectrum of a fluorescent marker of interest. The pulsed light beam is directionally propagated to illuminate a plurality of predetermined illumination points in a region of interest of the turbid media. Light emanating from a plurality of predetermined collection points in the region of interest is collected. The collected light includes a fluorescence signal from the fluorescent marker. The collected light is filtered to allow the fluorescence signal to propagate through a filter while rejecting photons outside a fluorescence emission spectrum of the fluorescent marker. The filtered light is measured at a detector to produce a time resolved optical signal for one or more illumination point/collection point configurations.

According to the present disclosure, there is also provided a system for collecting optical data for use in time resolved optical imaging of a turbid media. The system comprises a pulsed light source providing a light beam at a tunable wavelength according to an excitation wavelength of a fluorescent marker of interest. Also comprised is an illuminating optic component for directionally propagating the pulsed light beam such that a region of interest of the turbid media is illuminated at a plurality of illumination points. A collecting optic component collects light emanating from a plurality of predetermined collection points in the region of interest. The collected light includes a fluorescence signal from the fluorescent marker. A fluorescence filter allows the fluorescence signal to propagate therethrough while rejecting photons outside a fluorescence emission spectrum of the fluorescent marker. A time domain detector detects the filtered light and produces a time resolved optical signal for one or more illumination point/collection point configurations.

The foregoing and other features will become more apparent upon reading of the following non-restrictive description of illustrative embodiments thereof, given by way of example only with reference to the accompanying drawings.

### BRIEF DESCRIPTION OF THE DRAWINGS

Embodiments of the disclosure will be described by way of example only with reference to the accompanying drawings, in which:
Fig. 1 is a flow chart of an exemplary method for collecting optical data for use in time resolved optical imaging;
Fig. 2 is perspective view of an embodiment of a system for collecting optical data for use in time resolved optical imaging;
Fig. 3 is a detailed view of some of the optic components of Fig. 2;
Fig. 4 schematically illustrates a raster scan pattern of illumination in a region of interest at the surface of a mammal;
Fig. 5 is a block diagram of an example of imaging system; and
Figure 6 is a graphical representation of an example of laser wavelength and fluorescence filter optimization.
It will be noted that throughout the appended drawings, like features are identified by like reference numerals.

### DETAILED DESCRIPTION

Various aspects of the present disclosure generally address one or more needs related to optical imaging systems and methods supporting a diversity of fluorescent markers.

The present disclosure relates to the field of optical imaging of turbid media such as biological tissues as parts of human organs, animals, and the like. While the following description of exemplary embodiments provides examples that relate to imaging of small mammals such as mice, it will be appreciated that the method can also be applied in clinical testing for the benefit of actual patients as well as to laboratory testing involving larger animals and in particular to laboratory animals such as dogs, pigs and primates. US Patent No 6,992,762, describes a system and method for collecting optical data using one or more fixed wavelength light sources. Furthermore, the documents WO 2005/043138 A1, US 2008/0051665A1, and WO 2006/091223 A2 disclose systems and methods according to the preamble of the independent claims.

The following terminology is used throughout the present disclosure:
Time resolved imaging: Imaging based on time required by photons to travel within an object being imaged.
Time domain detector: A detector sensitive to timed characteristics of a signal.
Turbid media: A substantially opaque medium due to a relatively high light scattering, for example a biological tissue.
Region of interest (ROI): Part of a turbid media to be imaged.
Fluorescent marker: A molecule inserted into a turbid media, capable of emitting light when subject to energy transfer.
Biomarker: A biological or biologically-derived marker.
Fluorescence signal: Light emitted from a fluorescent marker.
Fluorescence spectrum: A wavelength, or a wavelength range, of light emitted by a fluorescent marker.
Excitation wavelength: Wavelength causing a fluorescent marker to emit light, usually at another wavelength distinct from the excitation wavelength.
Excitation spectrum: Range of excitation wavelength causing a fluorescent marker to emit light.
Tuning: Adjusting wavelength or frequency.
Pulsed light: Light emitted intermittently, according to a duty cycle.
Directionally propagating: Of light, emitting in a non-dispersive (collimated) fashion.
Illuminating optic component: Optical device on the emitting side of an illuminating system, located upstream of an illumination target.
Collecting optic component: Optical device on the receiving side of an illuminating system, located downstream of an illumination target.
Maximum rejection point: Of a filter, a wavelength at which rejection by the filter is maximized.
Fluorescence filter: A filter for a fluorescence signal.
Excitation filter: A filter located upstream of an illumination target.
Collection filter: A filter located downstream of an illumination target.
Free space optics: Line-of-sight optics in which light travels unimpeded through empty space, air, or like medium.
Controller: A device such as a computer or a processor capable of controlling another device or component based on an input.

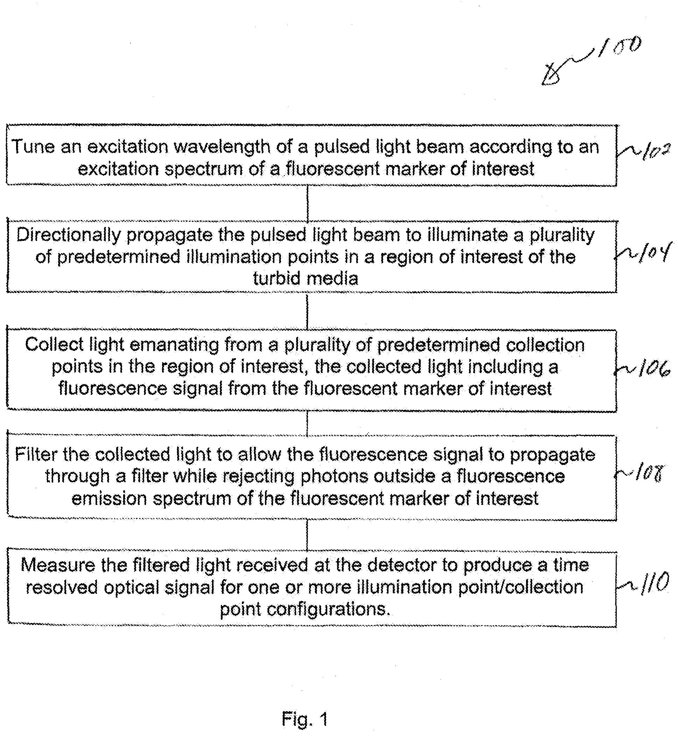

With reference to the drawings, Fig. 1 is a flow chart of an exemplary method for collecting optical data for use in time resolved optical imaging. Optical imaging of a turbid media, such as for example a biological tissue or a part of a human organ or animal organ, may be obtained using a sequence 100 comprising a first step 102 of tuning an excitation wavelength of a pulsed light beam according to an excitation spectrum of a fluorescent marker of interest. For example, a laser wavelength could be adjusted as close as possible to a wavelength for maximum absorption of Cy5.5 fluorescent probes (i.e. -675nm), while maintaining an optimum offset compared with a cut-on of the fluorescence filter for maximizing rejection of laser light. At step 104, the pulsed light beam is directionally propagated to illuminate a plurality of predetermined illumination points in a region of interest of the turbid media. Light emanating from a plurality of predetermined collection points in the region of interest is collected at step 106. The collected light includes a fluorescence signal from the fluorescent marker of interest, and may also contain other components such as for example fluorescence from tissue and light from the excitation laser beam. At step 108, the collected light is filtered to allow the fluorescence signal of interest to propagate through a filter while rejecting photons outside a fluorescence emission spectrum of the fluorescent marker of interest. The filtered light is measured at a detector, at step 110, to produce a time resolved optical signal for one or more illumination point/collection point configurations.

The emitted light, the collected light and the filtered light may propagate between various optical components in air (i.e. through free space optics) or through optical components such as fiber optics

The fluorescent marker of interest, present in the region of interest, may comprise one or more biomarkers, in which case the excitation wavelength may be tuned to correspond to an excitation wavelength of one or more biomarkers. As multiple fluorescent markers with different excitation wavelengths may be used concurrently or in different areas of the turbid medium, the excitation wavelength of the pulsed light beam may be tuned in various ways.

Tuning the excitation wavelength may be such that it matches the excitation spectrum of one or several fluorescent marker(s) of interest, or biomarker(s) either concurrently or sequentially. Variations of the tuning may be made in order to allow maximum rejection of the excitation wavelength by the filter while at the same time obtaining a sufficient fluorescence level. The filter, which may be a fluorescence filter, may be adaptable for maximizing a collection within a spectrum of a fluorescence signal of interest, corresponding to the fluorescent marker, while at the same time rejecting wavelengths that are not of interest. In particular, the fluorescence filter may minimize interference in the fluorescence signal of interest stemming from the excitation light source itself. Otherwise stated, the filter may be adapted to minimize leaking of the light emitted from the source, in its original wavelength, into the detector. Tuning may be performed manually or automatically. Information regarding a level of rejection by the filter may be fed back to a source of the pulsed light via a controller such as for example a computer (shown on a later figure). Additionally, an intensity of the pulsed light beam may be adjusted, for example based on one or several of the following: specifics of the turbid media, on geometry of various optical elements (shown on later figures) used in collecting the collecting optical data, or on an intensity of the collected light. Thus both the pulsed light beam and the filter are adjustable to allow efficient use with various fluorescent markers and biomarkers.

Light emanating from a plurality of collection points after diffusion through the tissue has a somewhat different, usually longer wavelength than that of the light emitted from the source. Some fluorescent markers, biomarkers, or fluorophores, may differ and fluoresce at a shorter wavelength than that of the light emitted from the source. The present disclosure is not limited to any type of fluorescent marker or biomarker and includes fluorescent wavelengths that are either longer or shorter than the excitation wavelengths.

The collected light measured using the detector is used to produce a time resolved optical signal. Light collection may be selective so that light emanating from points other than those being sampled may be optically excluded from detection.

Embodiments of the system used for collecting the optical data will now be described mostly referring to small mammals as the object to be imaged but it will be appreciated that a wide variety of biological tissues may be amenable to optical imaging using the technique described herein. These can be but are not limited to breast tissue, brain, tumors and the like.

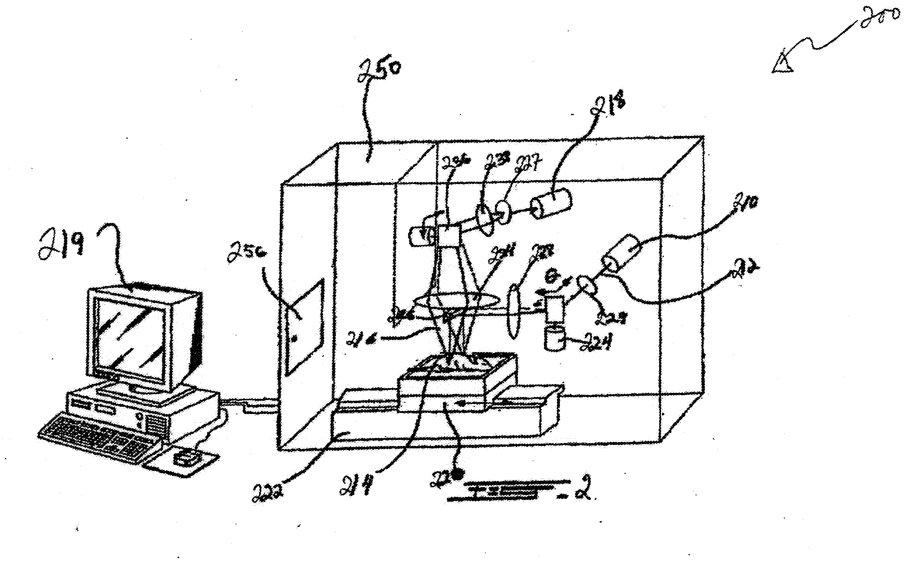

A general schematic representation of a first embodiment of a system used for imaging turbid media, represented as a small mammal, is shown in Fig. 2, which is an exemplary perspective view of an embodiment of a system for collecting optical data for use in time resolved optical imaging. The system 200 comprises a pulsed light source 210 providing a pulsed light beam 212 at a tunable wavelength. The wavelength of the pulsed light beam 212 may be tuned according to an excitation wavelength of a fluorescent marker of interest present in the turbid media being imaged 214.

The pulsed light source 210 is capable of generating a beam of light 212 at an adaptable or tunable wavelength. Illuminating optic components, comprising for example a movable reflective mirror 224, directionally propagate the pulsed light beam 212 such that a region of interest of the turbid media is illuminated at one or a plurality of predetermined illumination points. Collecting optic components, for example a collecting lens 234, collect light emanating from a plurality of predetermined collection points in the region of interest. The collected light includes a fluorescence signal from the fluorescent marker(s) or biomarker(s) of interest. A fluorescence filter allows the fluorescence signal(s) to propagate therethrough while rejecting photons outside a fluorescence emission spectrum of the fluorescent marker(s) of interest. In some embodiments, a single component forms the fluorescence filter. In a variant, the fluorescence filter may comprise an excitation filter 229 in a path of the light beam 212 and a collection filter 227 in a path of the collected light. Alternately, the collection filter 227 may consist of a plurality of filters to allow filtering of photons of wavelength other than the fluorescence emission spectrum of the fluorescence marker(s) and biomarker(s) of interest. A time domain detector 218 detects the collected light and if applicable filtered light and produces a time resolved optical signal for one or more illumination point/collection point configurations. A fluorescence lifetime of a biomarker refers to the average time the biomarker molecule stays in its excited state before emitting a photon. In an embodiment, post-processing of the information collected by the detector 218 may be made at the computer 219, based on a known fluorescence lifetime of a biomarker.

As shown on Fig. 2, illuminating optics may directionally propagate the beam of light through free space, toward desired illumination points on the surface of the turbid medium 214. Likewise, collecting optics may collect the light 216 re-emitted from the turbid medium through free space, forwarding the light through a collection filter 227 and further to the detector 218. Another embodiment of the system may use fiber optics (not shown) associated with various optic components, rather than free space optics, for at least some or all of the light paths of Fig. 2.

As shown a movable supporting tray 220 is mounted on a translational stage 222. A computer 219 is an exemplary controller that may be used for controlling the pulsed light source 210, the various optic components, the detector 218 and the tray 220.

The movable reflective mirror 224 may be a mirror galvanometer. The beam 212 may pass through the excitation filter 229 and then be reflected by the movable mirror 224 at an angle θ and directed towards a thin angled mirror 226 which reflects the beam in a direction substantially perpendicular to the surface of the turbid medium 214 being scanned. It can be appreciated that the partial rotation of the movable mirror 224 will modify the angle θ and direct the beam to a different point on the thin angled mirror 226 and, consequently, to a different illumination point on the surface of the turbid medium 214. Successive partial rotations of the movable mirror 224 thus produce a line scan substantially parallel to the thin angled mirror 226. Lens 228 is optionally provided and positioned between the movable mirror 224 and the thin angled mirror 226 such that the movable mirror 224 is at the focal distance of the lens 228 to provide telecentric imaging.

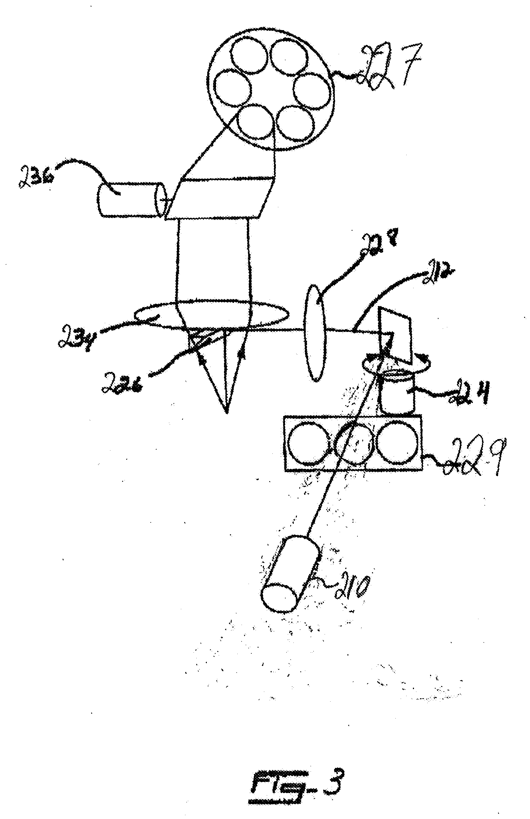

Fig. 3 is a detailed view of some of the optic components of Fig. 2, with additional components. The pulsed light source 210 emits light at a tunable wavelength and may do so at a variable intensity. The pulsed light source 210 may be a tunable laser, for example a supercontinuum tunable laser having a dispersion device (not shown) for selecting a desired wavelength or bandwidth. Using non-linear optical effects the supercontinuum laser is able to generate ultrashort light pulses (picoseconds) with a very broadband spectrum (from 400nm up 2400nm). A spectral selector with fixed or adjustable bandwidth is used to extract from this spectrum the optimum bandwidth and power required for the excitation of any fluorophore. Alternatively, the pulsed light source 210 may be a xenon lamp but its pulse duration is much longer (microseconds) and power density much lower limiting drastically the sensitivity, the range of fluorescence lifetimes that could be measured and the accuracy of the depth & concentrations (3D volumetric) evaluations by comparison with the supercontinuum laser.

The excitation filter 229 and the collection filter 227 are positioned, respectively, between the pulsed light source 210 and a region of interest (ROI), and between the re-emitted light and the detector 218 (shown on Figure 2), which may be a time correlated single photon counting detector. The movable mirror 224 may be a switching or dichroic mirror system and may be used for either sequential or simultaneous illumination of the ROI at different wavelengths. The excitation filter 229, the movable mirror 224 and the collection filter 227 together may form an adaptable fluorescence filter. In an embodiment, a selectable fluorescence filter may alternatively be used. The fluorescence filter rejects wavelengths outside the spectrum of fluorescence signal(s) of interest. The fluorescence filter may further have spectral regions of maximum rejection (for wavelengths outside those specific to the fluorophore of interest) where the collected light is rejected to a large extent (>6OD) while the fluorescence wavelengths of interest from the light reflected on the ROI is to a large extent unimpeded by the fluorescence filter. Of course, embodiments supporting phenomena in which detected fluorescent light having a shorter wavelength than an excitation wavelength are also within the scope of the present disclosure. Information about rejection, including about the maximum rejection point, may be fed back from the detector 218 to the pulsed light source 210. Observation and adaptation of this maximum rejection point may be used to adjust the tunable wavelength of the pulsed light source 210, thereby improving the rejection of the pulsed light source wavelength while maintaining optimum excitation efficiency.

In an embodiment, a user of the system may adjust the wavelength of the pulsed light source 210 manually, via observation of impacts of such adjustments on a behavior of the light reflected on the ROI. In another embodiment, the wavelength adjustment may be made automatically, under control of the computer 219, on the basis of feedback and/or detected light from the detector 218. Those of ordinary skill in the art will appreciate that feedback from the detector 218 about rejection of the excitation wavelength may be provided to the tunable pulsed light source 210 by the computer 219 and/or other types of controllers (not shown) by means of appropriate software and code.

Returning to Fig. 2, tray 220 supports the exemplary turbid medium 214, in the current graphical representation the mammal, while it is being imaged. The tray can be displaced longitudinally on a translational stage 222 to position the turbid medium such that a plurality of line scans parallel to each other can be generated. This stepwise process is repeated a selected number of times to produce a raster scan of a region of interest. The raster scan can alternatively be achieved by longitudinally displacing the thin angled mirror 226. The raster scan can be also generated by using fiber optics for illumination of the turbid medium and collection of the signal that are installed on the arm of a robot that can generate 3D movements following the shape of the turbid medium or specimen under investigation.

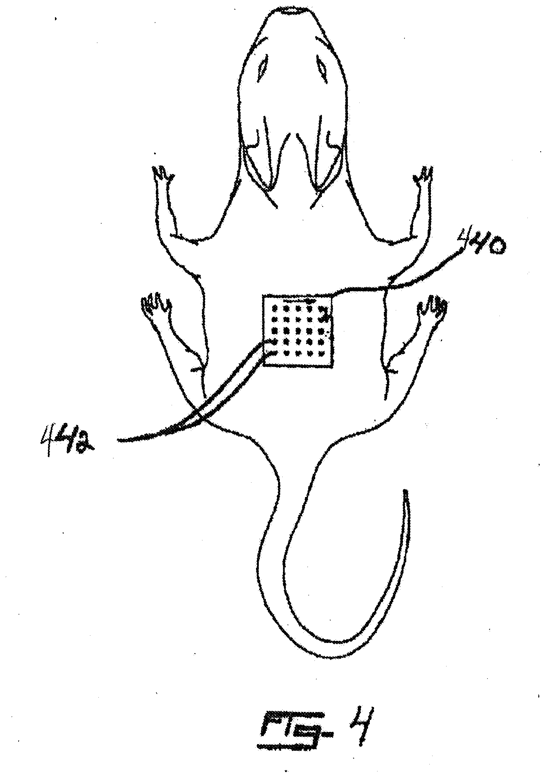

Fig. 4 schematically illustrates a raster scan pattern of illumination in a region of interest (ROI) at the surface of a mammal. The user defined ROI 440 delimits the area to be scanned which comprises the predetermined illumination points 442 according to a selected configuration. Predetermined collection points may generally correspond to the illumination points 442. The arrangement of the optic components also permits other scanning patterns to be performed. It will be appreciated that the ROI may consist of the whole animal.

Considering at once Figs. 2 and 3, light re-emitted from the turbid medium is collected by the collecting optics, which may comprise collecting lens 234 and may additionally comprise reflective mirror 236 which may be a mirror galvanometer, collection filter 227 and lens 238. The collecting lens 234 is located above the ROI and above the thin angled mirror 226. The angular position of the mirror 236 relative to the incoming light and the detector 218 determines which collection point is being sampled since only part of the light re-emitted (corresponding to a given collection point) impinging on the mirror is reflected at the proper angle to reach the detector 218. Selective detection of the light re-emitted from a given collection point may be further enhanced by optically coupling the mirror galvanometer with lenses and/or pinholes.

Upon impinging on the surface of the ROI, part of the excitation light penetrates the tissue and part is reflected at the air/tissue boundary. The photons of the excitation light that are propagated within the ROI are absorbed and scattered, thereby producing a large number of photon paths. In biological tissues, absorption may arise as a result of the presence of natural (endogenous) or exogenous chromophores, biomarkers or fluorescent markers, while scattering is triggered by the presence of micro and macromolecular structures such as cell nucleus and organelle, proteins, lipids and the like which create refractive index inhomogeneities. The fraction of the excitation light that is not absorbed ultimately exits the ROI by diffusing through the skin barrier at various distances from the illumination point. It can be appreciated that photons that have traveled deeper in the tissue will take a longer time to exit at the surface of the ROI. This provides the basis for time resolved detection of the collected light signal from which useful information about the optical properties of a region of interest can be extracted to be incorporated into image reconstruction algorithms. Throughout the present specification, time resolved and time domain (TD) are alternately used, but refer to the same principle. I

In TD measurements, the pulsed light source is briefly pulsed and the collected light signal is detected as a function of time to generate a temporal point spread function (TPSF). The light source may be a laser source capable of generating pulses characterized by a width in the picoseconds range. Time domain detectors such as time gated intensified charge coupled devices (ICCDs), time correlated single photon counting devices (TCSPC's), ultrafast semiconductor detectors (avalanche and PIN photodiodes), photomultipliers and streak cameras can be used. In an embodiment, a TCSPC device is used in the optical system. TCSPC's are capable of measuring the time taken by a photon to reach the detector as it travels through the illuminating optical path, the tissue and the collecting optical path. Time measurements may be provided by a "clock" circuitry electronically coupling the light source and the detector.

While the TD imaging of turbid medium can rely on the natural optical properties of the endogenous molecules for providing optical contrast, exogenous molecules may be introduced in the tissue to provide additional contrast. In this respect, exogenous chromophores as well as fluorophores and biomarkers may be used as contrast agents. Furthermore the biodistribution of such contrast agents can be followed using the method and system described hereinabove. In an embodiment, the biodistribution can be followed over time thereby producing pharmacokinetics data.

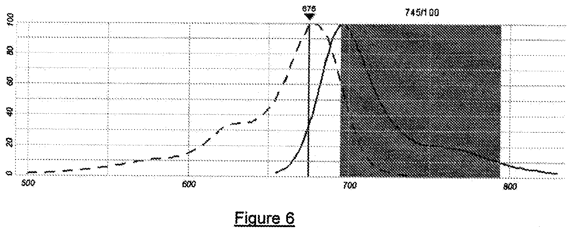

Reference is now also made to Figure 6, which depicts a graphical representation of an example of laser wavelength and fluorescence filter optimization. The various optical components as well as the light source are arranged to illuminate and detect light at a tunable wavelength, as is described hereinabove. This property can be exploited to follow the pharmacokinetics of two or more biomarkers such as fluorophores and/or chromophores. In particular, the tunable source may be arranged to illuminate at an excitation wavelength of a fluorophore while the fluorescence filter maintains optimum selectivity at an emission wavelength, or more generally in an emission spectrum, of the fluorophore. The optical components and the light source may thus be tuned to subsequently measure one or several fluorophore(s) and or chromophores(s), so as to extract combined and more elaborate pharmacokinetics data.

In addition to the formerly described components, the system may further comprise one or several of the following components. The selection of additional components depends on the applications, the type of turbid medium, the type of contrast agent(s), the pharmacokinetics data sought, etc. Thus the following components can be added separately, as sub-combinations or concurrently to the previously described system. Therefore, various embodiments of systems for collecting optical data for use in time resolved optical imaging, as disclosed herein, may be envisioned.

### System for Visible Image Capturing

The imaging system may comprise an illumination system having means for adjusting the illumination intensity and/or sensitivity. As an example, a charged coupled device (CCD) camera with adjustable sensitivity and contrast may allow easy discrimination between a turbid medium or specimen and a carrier used for the installation in the imaging area (background of the imaging area) for any expected pigmentation in order to realize an automated specimen location identification and scan only the turbid medium or specimen.

### Profilometer & 3D Raster Scanner

A profilometer providing laser power optimization according to optical properties of the turbid medium, may be used to allow maximum accuracy for the profile independent of visual physical characteristics of the turbid medium. An automatic three dimensional (3D) scan of the turbid medium minimizing the shape effect on the depth of field of the illumination and detection optics may be obtained.

### Illumination Subassembly

In yet another aspect, a multi-wavelength pulsed laser source may be used to provide a wide spectral coverage matching the requirements for the excitation of an extended number of fluorescent markers. Such laser source may be configured for a pulse of duration in the picoseconds range for providing a required temporal resolution. An option may be provided for selecting between multiple repetition rates for the pulses for allowing optimum efficiency when investigating fluorophores with very different lifetimes, for example from few hundred picoseconds to microseconds. The multi-wavelength pulsed laser source may be formed from a combination of many pulsed lasers with different wavelengths, a pulsed tunable laser that has internal means for wavelength selection, or a pulsed supercontinuum laser with a spectral selector that allow the selection of optimized narrow bandwidth. The spectral selector may be an acousto-optical tunable filter (AOTF), a dispersive prism based filter, interferential filters, or narrow band selective reflection mirrors. For the AOTF, additional spatial filtering may be performed for improving the spectral purity of the selected bandwidth. A pulse-picker may be included for flexible selection of the pulse repetition rate. A combined solution of using these sources may be used to acquire fluorescence data.

### Illumination system with multi-channel software controlled attenuators

Multi-channel software (SW) controlled attenuators may be used to provide means for independent excitation signal optimization for each of the selected wavelengths.

### Time-multiplexing module

A time-multiplexing module may be used to provide means for optimum and highly accurate temporal correlation between the selected wavelengths and the time-window (time-gate) of the detection channel. Temporal multiplexing-demultiplexing is used for separation of light signals generated by different wavelengths without requiring supplementary spectral demultiplexing. It is used for pump-probe experiments using controlled delay between the wavelengths used for initiating a process and respectively interrogating the status of the process at a certain moment after its initiation.

### Spectral multiplexing module

A spectral multiplexing module may further be used to provide means for spectral multiplexing of two or more wavelengths required for sequential or simultaneous excitation of multi-fluorescent markers cocktails.

### Illumination-detection optical channels

Illumination-detection optics may be installed on an arm of a robot to allow performing 3D raster scanning of the turbid medium. The configuration is adjustable so it also generates spot sizes and optimized separation required by constraints imposed by a mathematical model.

### Signal collection module

A signal collection module of the detection channel may be configured to perform sequential or simultaneously the collection of the re-emitted light from one or more well defined small areas (detection points) on the surface of the turbid medium.

### Spectral demultiplexer

A spectral demultiplexer is used to spectrally separate the collected light and transfer it to the detector(s). In various embodiments, it may comprise interference filter(s), an acousto-optical tunable filter, or dispersive elements such as prisms or gratings, as non-limiting examples. Sequential transfer may be made towards a single detector. Alternatively, parallel transfer may be made towards multiple detectors or an array of detectors.

### Detection system with multi-channel software controlled attenuators

Multi-channel SW controlled attenuators may further be used with the present system to provide means for independent optimization of the level of the collected light for each of the selected spectral band to compensate the differences in overall efficiency of the fluorescent markers that are excited-detected simultaneously.

### 3D raster scanning module

A high precision 3D raster scanning module and robot with 3 axes may be used for obtaining 3D raster scan. The 3D raster scanning module provides means for installing an illumination and detection head in the configuration required by a mathematical model used (optimized separation between the illumination and detection spots). The method may further comprise a flexible scanning step which allows the selection of the image resolution that matches better the needs.

### Self-diagnostic module

A self-diagnostic module is used to provide means for system diagnostic using the following sequence: validating the functionality of each components and module, validating the illumination subsystem status, and validating the whole collection/acquisition system status. The results of the whole system status are used as input for corrections of the data for compensating changes generated by aging of some of the components or unexpected environmental changes.

### Specimen table

Various types of specimen tables from dedicated carriers may be used for the samples/ turbid medium to be investigated. These tables may include, as non-limiting examples, vials support, well-plates table, small animal supports for a single animal or for up to five (5) animals simultaneously, or an isolation box. Multi-modality imaging may take the form of Optical-CT, Optical-MRI, or Optical-PET. These carriers are provided with means for animal control, care and monitoring that may include means for controlled temperature, for anesthetic and oxygen delivery, and for animal positioning monitoring.

### System control and data acquisition

A system control and data acquisition module may be used in an interactive loop for analyzing the quality of the image and making required adjustments, for optimizing the illumination level and CCD sensitivity, for improved contrast of the turbid medium (for example performance less dependent on its pigmentation, easier to discriminate when using "whole specimen" option during acquisition, possibility to implement an automated process instead of manual drawing of the ROI, etc.). The system control and data acquisition module may be installed for example in combination with a carrier that imposes a certain positioning for the installation of the turbid medium.

The system control and data acquisition module provides means for visualizing and selecting regions of interest according to the goal of the study, for example based on visible image of the turbid medium/specimen, visible image of the specimen in conjunction with preliminary quality parameters features of the preliminary image or an anterior study-image.

This system control and data acquisition module further provides means for optimizing the workflow according to the specificity of the study, for each of the typical phase of the process. This applies to various types of turbid media, such as *in-vitro, in-vivo,* and *ex-vivo.* This further applies to various types of experiments. Supported experiments include Single fluorescent marker, such as using pre-defined wavelength & fluorescence filter configuration or using search loop for optimum excitation & collection efficiencies when using continuously adjustable wavelength selector for the laser and spectral demultiplexer for the detection at optimized central wavelength, bandwidths and offset between the cutoff and cut-on of the two bandwidths, or optimized wavelengths for maximizing the brightness in the cases of multi-photon processes (as Up-converting nanoparticles, etc.). Other supported experiments include multi-fluorescent labels experiments, such as excitation & detection of multiple probes/markers attached to same or multiple biomarkers allowing higher throughput experiments and increased accuracy validation assay, and Pump-probe experiments performed by generating the required combination of wavelengths for initiating the process and interrogation of its status.

This system control and data acquisition module is amenable to various ROI sizes, patterns and step sizes, various levels of data quality in terms of intensity, including full quantitative, various acquisition sequences including single-scan and multiple-scans using pre-defined delay between scans.

The system control and data acquisition module provides means for automated optimization of the quality of the data acquired based on specific algorithms and criteria, for example regarding ROI segment wise or pixel wise optimization of the excitation power and integration time, in which pixels will have a signal to noise ratio (SNR) at least equal or superior to a minimum required by post-processing algorithms. The system control and data acquisition module is further usable for monitoring and saving useful parameters required for system monitoring and troubleshooting, and for collecting and saving parameters that are useful for data analysis.

### Data analysis and display

A data analysis and display module is used to provide means for data analysis in accordance with some mathematical algorithms and quality criteria and display of the characteristic parameters defined as quality features for the study. The list of the parameter includes for example an intensity map, fluorescence lifetime(s) for single or multiple fluorescent probes/markers, depth & concentration two dimensional (2D) image information, and 3D tomographic views of the fluorescent probe/marker, including 3D volumetric estimations.

The data analysis and display module may export data for further processing and multi-modality imaging co-registration, and provide means for high throughput data processing using list of user selectable tasks.

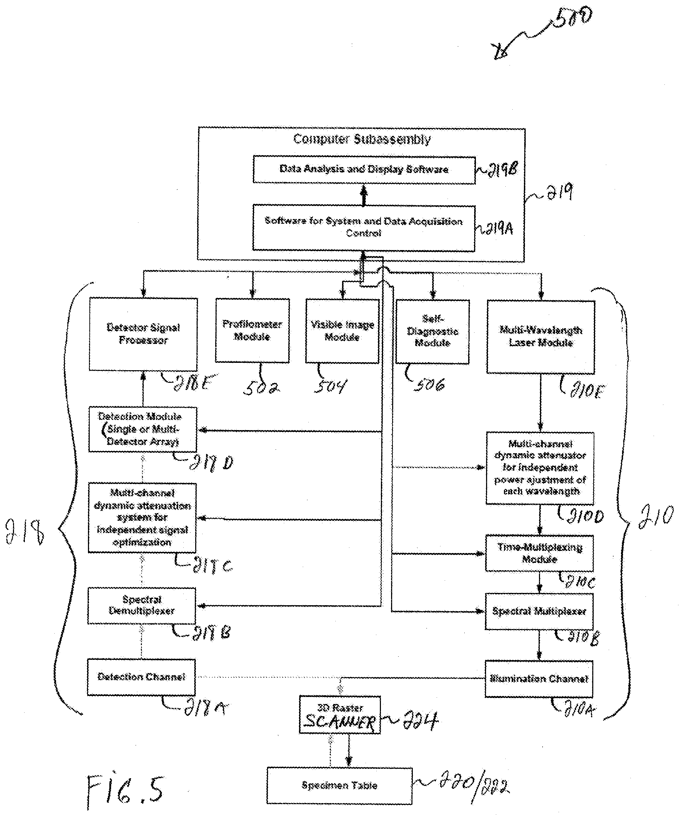

Fig. 5 is a block diagram of an example of an imaging system 500. The imaging system 500 as shown comprises optional variants of some of the components introduced hereinbefore. The imaging system 500 comprises the pulsed light source 210, the time domain detector 218, the computer 219, the supporting tray 220 in combination with the translational stage 222 and the movable mirror 224 introduced in the foregoing description of Figs. 2 and 3. Additional modules are added, including a profilometer module 502, a visible image module 504 and a self-diagnostic module 506.

The computer 219 as illustrated on Fig. 5 is functionally subdivided into two main components, comprising a software module and system for data acquisition control 219A, and a data analysis and display software module 219B. The software module and system for data acquisition control 219A provides control for operation of the imaging system 500, for example by using feedback information from the time domain detector 218 to control the pulsed light source 210. The software module and system for data acquisition control 219A further controls the modules 502-506. The data analysis and display software module 219B provides and displays information about optical images obtained from the turbid media.

The light source 210 may be functionally subdivided into various components. In an embodiment, the light source 210 comprises an illumination channel 210A, a spectral multiplexer 210B, a time-multiplexing module 210C, a multi-channel dynamic attenuator 210D capable of independently making power adjustments to each of a plurality of wavelengths, and a multi-wavelength laser module 210E. Building the light source 210 from the modules 210A-210E provides a laser source capable of rapidly acquiring an optical image of a turbid media, using a plurality of fluorescent markers subject to a variety of excitation wavelengths. Of course, a simpler tunable light source 210 may be used in other embodiments. Various rearrangements and modifications may be performed to the sub-components 210A-210E of the light source 210.

The time domain detector 218 may also be functionally subdivided into various components. It may comprise a detection channel 218A, a spectral demultiplexer 218B, a multi-channel dynamic attenuation system 218C capable of independently optimizing signals at a plurality of wavelengths, a single detection module 218D or a module 218D formed from an array of detectors, and a signal processor 218E. Various embodiments of the time domain detector 218 may comprise all or a subset of the modules 218A-218D.

Arrows on Fig. 5 show paths for information signals and for control signals. The computer 219 - or a suitable controller - is generally in control of the imaging system 500. It controls the tuning of the light source 210 and may do so using feedback from the time domain detector 218. The computer 219 also controls angular movements of the movable mirror 224, as well as movements of the supporting tray 220 in combination with the translational stage 222, in order to provide a 3D scanning of a plurality of predetermined collection points in a region of interest of the turbid media.

The systems and method introduced in the present disclosure involve a time-domain platform that offers maximum spectral coverage adapted to most of the known fluorescent probes/markers. High temporal resolution and low time jitter enable maximum accuracy for fluorescence lifetime evaluation, depth and concentration estimation, in 3D tomographic views. Highest flexibility is available for selecting and tuning the wavelength for maximizing the efficacy of excitation and detection as well as overall sensitivity for any fluorescent probe. Temporal and spectral multiplexing-demultiplexing are allowed for performing experiments capable of simultaneously testing a large number of biomarkers and design high throughput assays. Pump-probe experiments are made possible by multiplexing both spectral and temporal the wavelengths required for initiation of a process and monitoring its evolution by interrogating its status at predefined moments in time at ultra short time scales. Self-diagnostic is available in real-time. ROI and pixel-wise signal conditioning are capable of providing the level of data quality and signal to noise ratio (SNR) required for a highly accurate estimation by the model based algorithms used for data analysis. Data acquisition optimization is for best matching the assumptions and requirements of the mathematical models used for analysis and interpretation. Structured functionalities allow maximum flexibility in defining the optimum sequence and workflow for a given study.

Those of ordinary skill in the art will realize that the description of the systems and their sub-components and method for collecting optical data for use in time resolved optical imaging of a turbid media are illustrative only and are not intended to be in any way limiting. Other embodiments will readily suggest themselves to such persons with ordinary skill in the art having the benefit of the present disclosure. Furthermore, the disclosed systems and methods may be customized to offer valuable solutions to existing needs and problems of optical imaging.

In the interest of clarity, not all of the routine features of the implementations of the systems and method for collecting optical data for use in time resolved optical imaging of a turbid media are shown and described. It will, of course, be appreciated that in the development of any such actual implementation of the system and method described herein numerous implementation-specific decisions may need to be made in order to achieve the developer's specific goals, such as compliance with application-, system - and business-related constraints, and that these specific goals will vary from one implementation to another and from one developer to another. Moreover, it will be appreciated that a development effort might be complex and time-consuming, but would nevertheless be a routine undertaking of engineering for those of ordinary skill in the field of optical imaging having the benefit of the present disclosure.

Systems and modules described herein may comprise software, firmware, hardware, or any combination(s) of software, firmware, or hardware suitable for the purposes described herein. Software and other modules may reside on servers, workstations, personal computers, computerized tablets, personal digital assistants (PDA), and other devices suitable for the purposes described herein.

It is to be understood that the present disclosure is not limited in its application to the details of construction and parts illustrated in the accompanying drawings and described hereinabove. The present disclosure is capable of other embodiments and of being practiced in various ways. It is also to be understood that the phraseology or terminology used herein is for the purpose of description and not limitation. Hence, although the present disclosure has been described hereinabove by way of illustrative embodiments thereof, it can be modified, without departing from the scope and nature of the present disclosure.

## Claims

1. A method for collecting optical data for use in time resolved optical imaging of a turbid media, the method comprising:
tuning an excitation wavelength of a pulsed light beam of a pulsed light source according to an excitation spectrum of a fluorescent marker of interest (102);
directionally propagating the pulsed light beam to illuminate a plurality of predetermined illumination points in a region of interest of the turbid media (104);
collecting light emanating from a plurality of predetermined collection points in the region of interest, the collected light including a fluorescence signal from the fluorescent marker (106);
filtering the collected light to allow the fluorescence signal to propagate through a filter while rejecting photons outside a fluorescence emission spectrum of the fluorescent marker (108) and
measuring the filtered light at a detector to produce a time resolved optical signal for one or more illumination point/collection point configurations (110),
**characterized by** tuning the excitation wavelength based on a rejection of the excitation wavelength by the filter by feeding back information regarding a level of rejection by the filter from the detector to the pulsed light source.

2. The method as claimed in claim 1, wherein the excitation wavelength is tuned based on a maximum rejection point of the filter.

3. The method as claimed in any of claims 1 - 2, further comprising adjusting an intensity of the pulsed light beam.

4. The method as claimed in any of claims 1 - 3, wherein the region of interest comprises one or more biomarkers and wherein the excitation wavelength is tuned to correspond to an excitation wavelength of the one or more biomarkers.

5. A system adapted to collect optical data for use in time resolved optical imaging of a turbid media, the system comprising:
a pulsed light source (210) for providing a light beam at a tunable wavelength according to an excitation wavelength of a fluorescent marker of interest;
an illuminating optic component (224) for directionally propagating the pulsed light beam such that a region of interest of the turbid media is illuminated at a plurality of illumination points;
a collecting optic component (234) for collecting light emanating from a plurality of predetermined collection points in the region of interest, the collected light including a fluorescence signal from the fluorescent marker;
a fluorescence filter (224, 227, 229) adaptable for allowing the fluorescence signal to propagate therethrough while rejecting photons outside a fluorescence emission spectrum of the fluorescent marker; and
a time domain detector (218) for detecting the filtered light and for producing a time resolved optical signal for one or more illumination point/collection point configurations,
**characterized by** a controller (219) for tuning the excitation wavelength of the pulsed light source based on a rejection of the excitation wavelength by the fluorescence filter by feeding back the information regarding a level of rejection of by the fluorescence filter from the time domain detector to the pulsed light source.

6. The system as claimed in any of claims 5, wherein the controller is further for tuning the excitation wavelength based on a maximum rejection point of the fluorescence filter.

7. The system as claimed in any of claims claim 5 - 6, wherein the light source is a variable intensity light source.

8. The system as claimed in any of claims 5 - 7, wherein the light source is a tunable laser.

9. The system as claimed in claim 8, wherein the tunable laser is a supercontinuum laser.

10. The system as claimed in claim 9, wherein the supercontinuum laser comprises a dispersion device for selecting the excitation wavelength from a supercontinuum spectrum.

11. The system as claimed in any of claims 5 - 10, wherein the time domain detector is a time correlated single photon counting detector.

12. The system as claimed in any of claims 5 - 11, further comprising one or several of the following: a system for visible image capturing, a profilometer, a 3D raster scanner, an illumination subassembly, an illumination system with multi-channel software controlled attenuators, a time-multiplexing module, a spectral multiplexing module, an illumination-detection optical channels, a signal collection module, a spectral demultiplexer, a detection system with multi-channel software controlled attenuators, a self-diagnostic module, and a system control and data acquisition.

## Patentansprüche

1. Verfahren zum Sammeln optischer Daten zur Verwendung bei einer zeitaufgelösten optischen Bildgebung eines trüben Mediums, wobei das Verfahren aufweist:
- Abstimmen einer Anregungswellenlänge eines gepulsten Lichtstrahles einer Quelle für gepulstes Licht entsprechend einem Anregungsspektrums eines Fluoreszenz-Markers von Interesse (102);
- direktionales Ausbreiten des gepulsten Lichtstrahles, um eine Mehrzahl vorbestimmter Beleuchtungspunkte in einer Region von Interesse des trüben Mediums (104) zu beleuchten;
- Sammeln von Licht, das von einer Mehrzahl vorbestimmter Sammelpunkte in der Region von Interesse ausstrahlt, wobei das gesammelte Licht ein Fluoreszenzsignal von dem Fluoreszenz-Marker (106) umfasst;
- Filtern des gesammelten Lichtes, um dem Fluoreszenzsignal zu ermöglichen, sich durch ein Filter auszubreiten, während Photonen außerhalb eines Fluoreszenzimmissionsspektrums des Fluoreszenz-Markers (108) ausgesondert werden; und
- Messen des gefilterten Lichtes an einem Detektor zum Erzeugen eines zeitaufgelösten optischen Signals für einen oder mehrere Beleuchtungspunktkonfigurationen (110)/Sammelpunktkonfigurationen (110);
- **gekennzeichnet durch** Abstimmen der Anregungswellenlänge auf Grundlage eines Aussonderns der Anregungswellenlänge durch das Filter, in dem eine Information bezüglich eines Niveaus des Aussonderns durch das Filter von dem Detektor an die gepulste Lichtquelle zurückgekoppelt wird.

2. Verfahren nach Anspruch 1, wobei die Anregungswellenlänge auf Grundlage eines Punktes maximalen Aussonderns des Filters abgestimmt wird.

3. Verfahren nach Anspruch 1 oder 2, ferner aufweisend das Anpassen einer Intensität des geputzten Lichtstrahles.

4. Verfahren nach einem der Ansprüche 1 bis 3, wobei die Region von Interesse einen oder mehrere Bio-Marker aufweist und wobei die Anregungswellenlänge so abgestimmt wird, dass sie einer Anregungswellenlänge eines oder mehrerer Bio-Marker entspricht.

5. System, das zum Sammeln optischer Daten zur Verwendung bei einer zeitlich aufgelösten optischen Bildgebung eines trüben Mediums ausgebildet ist, wobei das System umfasst:
- eine gepulste Lichtquelle (210) zum Bereitstellen eines Lichtstrahls bei einer abstimmbaren Wellenlänge gemäß einer Anregungswellenlänge eines Fluoreszenz-Markers von Interesse;
- eine optische Beleuchtungskomponente (224) zum direktionalen Ausbreiten des gepulsten Lichtstrahls, so dass eine Region von Interesse des trüben Mediums bei einer Mehrzahl von Beleuchtungspunkten beleuchtet wird;
- eine sammelnde optische Komponente (234) zum Sammeln von Licht, das von einer Mehrzahl von vorbestimmten Sammelpunkten in der Region von Interesse ausstrahlt, wobei das gesammelte Licht ein Fluoreszenzsignal von dem Fluoreszenz-Marker aufweist;
- ein Fluoreszenzfilter (224, 227, 229), das dazu anpassbar ist, um dem Fluoreszenzsignal zu ermöglichen, sich dadurch auszubreiten, während Photonen außerhalb eines Fluoreszenzemissionsspektrums des Fluoreszenz-Markers ausgesondert werden; und
- einen Zeitdomänendetektor (218) zum Detektieren des gefilterten Lichtes und zum Erzeugen eines zeitlich aufgelösten optischen Signals für eine oder mehrere Beleuchtungspunktkonfigurationen/Sammelpunktkonfigurationen,
- **gekennzeichnet durch** eine Steuerungseinrichtung (219) zum Abstimmen der Anregungswellenlänge der gepulsten Lichtquelle auf Grundlage eines Aussonderns der Anregungswellenlänge durch das Fluoreszenzfilter durch Rückkoppeln der Information bezüglich eines Niveaus des Aussonderns des Fluoreszenzfilters von dem Zeitdomänendetektor zu der gepulsten Lichtquelle.

6. System nach Anspruch 5, wobei die Steuerungseinrichtung ferner zum Abstimmen der Anregungswellenlänge basierend auf einem Punkt maximaler Zurückweisung des Fluoreszenzfilters eingerichtet ist.

7. System nach Anspruch 5 oder 6, wobei die Lichtquelle eine Lichtquelle variabler Intensität ist.

8. System nach Anspruch 5 bis 7, wobei die Lichtquelle ein abstimmbarer Laser ist.

9. System nach Anspruch 8, wobei der abstimmbare Laser ein Superkontinuumlaser ist.

10. System nach Anspruch 9, wobei der Superkontinuumlaser eine Dispersionseinrichtung zum Auswählen der Anregungswellenlänge von einem Superkontinuumsspektrum aufweist.

11. System nach einem der Ansprüche 5 bis 10, wobei der Zeitdomänedetektor ein zeitlich korrelierter Einzelphotonzähldetektor ist.

12. System nach einem der Ansprüche 5 bis 11, ferner aufweisend eines oder mehrerer des Folgenden: ein System zum Erfassen eines sichtbaren Bildes, eine Profilmesseinrichtung, ein 3D-Rasterscanner, eine Beleuchtungsunteranordnung, ein Beleuchtungssystem mit Dämpfern, die von einer Mehrkanalsoftware gesteuert werden, ein Zeit-Multiplexmodul, ein Spektral-Multiplexmodul, optische Kanäle zum Detektieren von Beleuchtung, ein einzelnes Sammelmodul, einen Spektral-Demultiplexer, ein Detektionssystem mit Dämpfern, die durch eine Mehrkanalsoftware gesteuert werden, ein Selbstdiagnosemodul und ein System zum Steuern und Erfassen von Daten.

## Revendications

1. Procédé pour collecter des données optiques pour une utilisation au niveau d'une imagerie optique à résolution temporelle d'un milieu turbide, le procédé comprenant :
l'accord d'une longueur d'onde d'excitation d'un faisceau de lumière pulsée d'une source de lumière pulsée conformément à un spectre d'excitation d'un marqueur fluorescent d'intérêt (102) ;
la propagation directionnelle du faisceau de lumière pulsée de manière à ce qu'il éclaire une pluralité de points d'éclairage prédéterminés dans une région d'intérêt du milieu turbide (104) ;
la collecte de la lumière qui émane d'une pluralité de points de collecte prédéterminés dans la région d'intérêt, la lumière collectée incluant un signal de fluorescence en provenance du marqueur fluorescent (106) ;
le filtrage de la lumière collectée de manière à permettre la propagation du signal de fluorescence au travers d'un filtre tout en soumettant à réjection des photons à l'extérieur d'un spectre d'émission de fluorescence du marqueur fluorescent (108) ; et
la mesure de la lumière filtrée au niveau d'un détecteur de manière à produire un signal optique à résolution temporelle pour une ou plusieurs configuration(s) de points d'éclairage/points de collecte (110) ;
**caractérisé par** l'accord de la longueur d'onde d'excitation sur la base d'une réjection de la longueur d'onde d'excitation par le filtre en retournant une information qui concerne un niveau de réjection par le filtre depuis le détecteur jusqu'à la source de lumière puisée.

2. Procédé tel que revendiqué selon la revendication 1, dans lequel la longueur d'onde d'excitation est accordée sur la base d'un point de réjection maximum du filtre.

3. Procédé tel que revendiqué selon l'une quelconque des revendications 1 et 2, comprenant en outre le réglage d'une intensité du faisceau de lumière puisée.

4. Procédé tel que revendiqué selon l'une quelconque des revendications 1 à 3, dans lequel la région d'intérêt comprend un ou plusieurs biomarqueur(s) et dans lequel la longueur d'onde d'excitation est accordée de manière à ce qu'elle corresponde à une longueur d'onde d'excitation des un ou plusieurs biomarqueurs.

5. Système adapté pour collecter des données optiques pour une utilisation au niveau d'une imagerie optique à résolution temporelle d'un milieu turbide, le système comprenant :
une source de lumière pulsée (210) pour produire un faisceau de lumière à une longueur d'onde accordable conformément à une longueur d'onde d'excitation d'un marqueur fluorescent d'intérêt ;
un composant optique d'éclairage (224) pour propager directionnellement le faisceau de lumière pulsée de telle sorte qu'une région d'intérêt du milieu turbide soit éclairée au niveau d'une pluralité de points d'éclairage ;
un composant optique de collecte (234) pour collecter la lumière qui émane d'une pluralité de points de collecte prédéterminés dans la région d'intérêt, la lumière collectée incluant un signal de fluorescence en provenance du marqueur fluorescent ;
un filtre de fluorescence (224, 227, 229) qui peut être adapté pour permettre la propagation du signal de fluorescence au travers tout en soumettant à réjection des photons à l'extérieur d'un spectre d'émission de fluorescence du marqueur fluorescent ; et
un détecteur de domaine temporel (218) pour détecter la lumière filtrée et pour produire un signal optique à résolution temporelle pour une ou plusieurs configuration(s) de points d'éclairage/points de collecte ;
**caractérisé par** un contrôleur (219) pour accorder la longueur d'onde d'excitation de la source de lumière pulsée sur la base d'une réjection de la longueur d'onde d'excitation par le filtre de fluorescence en retournant l'information qui concerne un niveau de réjection par le filtre de fluorescence depuis le détecteur de domaine temporel jusqu'à la source de lumière puisée.

6. Système tel que revendiqué selon la revendication 5, dans lequel le contrôleur est en outre prévu pour accorder la longueur d'onde d'excitation sur la base d'un point de réjection maximum du filtre de fluorescence.

7. Système tel que revendiqué selon l'une quelconque des revendications 5 et 6, dans lequel la source de lumière est une source de lumière d'intensité variable.

8. Système tel que revendiqué selon l'une quelconque des revendications 5 à 7, dans lequel la source de lumière est un laser accordable.

9. Système tel que revendiqué selon la revendication 8, dans lequel le laser accordable est un laser supercontinuum.

10. Système tel que revendiqué selon la revendication 9, dans lequel le laser supercontinuum comprend un dispositif de dispersion pour sélectionner la longueur d'onde d'excitation à partir d'un spectre de supercontinuum.

11. Système tel que revendiqué selon l'une quelconque des revendications 5 à 10, dans lequel le détecteur de domaine temporel est un détecteur de comptage de photons uniques à corrélation temporelle.

12. Système tel que revendiqué selon l'une quelconque des revendications 5 à 11, comprenant en outre un ou plusieurs des moyens qui suivent : un système pour capturer une image visible, un profilomètre, un scanner à balayage tramé 3D, un sous-assemblage d'éclairage, un système d'éclairage avec des atténuateurs commandés par logiciel à multiples canaux, un module de multiplexage temporel, un module de multiplexage spectral, des canaux optiques de détection d'éclairage, un module de collecte de signal, un démultiplexeur spectral, un système de détection avec des atténuateurs commandés par logiciel à multiples canaux, un module d'auto-diagnostic et un moyen de commande système et d'acquisition de données.
